# EUROPEAN PATENT APPLICATION

(11) **EP 2 977 458 A1**
(43) Date of publication of application: **27.01.2016**
(21) Application number: 14002560.2
(22) Date of filing: 23.07.2014
(51) Int. Cl.: C12N 15/82

(54) **Improved strains of Agrobacterium tumefaciens for transferring DNA into plants**

(71) Applicant: Rheinische Friedrich-Wilhelms-Universität Bonn, 53113 Bonn (DE)
(72) Inventor: Ülker, Bekir, 53115 Bonn (DE); Berson, Tobias, 53111 Bonn (DE)
(74) Representative: Krauss, Jan

(57) **Abstract**

The present invention relates to *Agrobacterium tumefaciens* strains that comprise at least one deletion/mutation in a sequence selected from the group of IS426 copy I, IS426 copy II, the *OriT*-like sequence, and the border-like sequences, and their uses in safer and improved transformation procedures for cells.

## Description

The present invention relates to *Agrobacterium tumefaciens* strains that comprise at least one deletion/mutation in a sequence selected from the group of IS426 copy I, IS426 copy II, the *OriT*-like sequence, and border-like sequences, and their uses in safer and improved transformation procedures for cells.

### Background of the invention

*Agrobacterium tumefaciens* is the workhorse of plant molecular biology and plant genetic engineering as this bacterium can efficiently transform plants. Methods using the bacterium have also been successfully used in transforming numerous other organisms, including human cells. However, as was demonstrated in 2008, the available *Agrobacterium* strains have hidden biosecurity risks.

In addition to the DNA of interest (i.e. to be transformed) contained within the T-DNA, sometimes very large other fragments of bacterial chromosomal DNA (AchrDNA) are also unintentionally transferred from the bacteria into plants (Ülker et al., 2008). Thus, besides the well-documented integration of DNA flanked by the transfer DNA borders, occasional insertion of fragments from the tumor-inducing plasmid into plant genomes has also been reported during *Agrobacterium tumefaciens*-mediated transformation. Large (up to 18 kb) gene-bearing fragments of *Agrobacterium* chromosomal DNA (AchrDNA) can be integrated into, for example, *Arabidopsis thaliana* genomic DNA during transformation. About one in every 250 transgenic plants may carry AchrDNA fragments. This has implications for horizontal gene transfer and indicates a need for greater scrutiny of transgenic plants for undesired bacterial DNA, as *Agrobacterium tumefaciens* still is a soil-borne bacterial pathogen of plants.

In nature, *Agrobacterium* transfers a defined segment of the tumor inducing (Ti) plasmid (T-DNA) into the host, leading to the formation of crown gall tumors controlled by T-DNA-encoded oncogenes. *Agrobacterium*-mediated DNA transfer has been exploited to introduce transgenes into plants and to transform other organisms such as yeast, fungi and even human cells. Sometimes, part of the Ti plasmid outside the T-DNA borders may be integrated into plant genomes. The *A. tumefaciens* strain C58 genome of 5.7 megabases has been completely sequenced and comprises four replicons: a linear chromosome, a circular chromosome and the two large plasmids AtC58 and TiC58.

Until the experiments that led to the present patent application, the mechanisms of how these chromosomal DNAs are transferred from bacteria to plants were not known. The present inventors have now determined the key mechanisms involved in this process.

It is therefore an object of the present invention to provide biosafe *Agrobacterium* strains that are less prone to the undesired transfer of DNA as described above. Furthermore, methods for improved transfer of DNA shall be developed. Other objects and advantages of the present invention will become apparent to the person of skill upon studying the following description of the present invention.

According to a first aspect of the present invention, the object is solved by providing an *Agrobacterium tumefaciens* strain, comprising at least one deletion and or mutation functionally inactivating said sequence in a sequence selected from the group of IS426 copy I, IS426 copy II, the *OriT*-like sequence, and the border-like sequences, for example the left- (*LB*) or right-border (*RB*) -like sequences. Preferred is at least one deletion.

The sequences to be mutated or deleted can also be selected from sequences with a nucleotide sequence that is at least 80%, more preferably, 90%, even more preferably 95% or 98% or 99% identical to the sequence of IS426 copy I, IS426 copy II, the *OriT*-like sequence, or the border-like sequences, for example the left- (*LB*) or right-border (*RB*) -like sequences, such as, for example, a sequence according to SEQ ID No. 1, 2, 3, 7, 8, 9 or 10.

Preferred is the *Agrobacterium tumefaciens* strain according to the present invention, wherein said strain comprises said deletion and/or inactivating mutation in two, three, or all four of said sequences.

An inactivating mutation in the context of the present invention shall mean a mutation that, when introduced into the elements as described herein, reduces, substantially reduces, or even abolishes the undesired transfer of DNA as described herein. Such mutation can be selected from a point mutation, but also includes several point mutations and/or added nucleotides for inactivation.

Further preferred is the *Agrobacterium tumefaciens* strain according to the present invention, wherein said deletion of said sequence is partially or fully, such as, for example, 30 bp in the *RB*-like sequence, and/or 61 bp in the *OriT*-like sequence.

Further preferred is the *Agrobacterium tumefaciens* strain according to the present invention, wherein the *OriT*-like sequence is located in the *HS1_{LC}* region and the *RB*-like sequence is located in the *HS1*_{CC} region.

As mentioned above, *Agrobacterium tumefaciens* is the workhorse of plant molecular biology and genetic engineering, as this bacterium can efficiently transform plants, which gave rise to the *Agrobacterium*-mediated transformation methods that have been the methods of choice when transforming plants. Numerous commercial transgenic crops generated using this technology are cultivated in several countries and are used in food, feeding or other industries. The methods have also been successfully used in transforming other organisms including human cells. *Agrobacterium* is a paradigm model for TypeIV SS employed by many human pathogenes such as *Helicobacter, Bartonella* and *Legionella. Agrobacterium*-mediated plant transformation is also the method of choice in most cases because it is a simple procedure, produces a high transformation efficiency, most plant species can be transformed, requires only a low transgene copy number, and can be done in basically every laboratory (S1).

While characterizing a T-DNA insertion locus named PM within the fully sequenced *A. thaliana* genome, the inventors discovered a 322-bp DNA fragment of non-plant origin associated with the right border (RB) of the T-DNA. The finding that this sequence is identical to a region on the sequenced linear chromosome of *A. tumefaciens* led the inventors to determine, whether this was a unique event or whether it is an intrinsic property associated with T-DNA transfer in general. Therefore, the inventors analyzed databases that contain *A. thaliana-*flanking sequence tags (FSTs), the sequences that flank T-DNA insertion sites in populations of insertion lines generated to saturate the genome with mutations. Fragments of AchrDNAs were detected in all tested T-DNA insertion databases, and AchrDNAs were found much more frequently in FSTs recovered from the RB. Based on these data, obtained from >375,000 T-DNA-tagged *A. thaliana* lines, the inventors estimated that about 0.4% (from the RB FSTs of GABI-Kat) of the insertion sites actually contain bacterial chromosomal DNA. The different populations as studied had been generated with different T-DNA vectors and A. *tumefaciens* strains, indicating that fragments of *A*chrDNA are transferred to the plant genome irrespective of the binary vector or *A. tumefaciens* strain used. In addition, the inventors also studied rice FST collections and detected AchrDNA sequences, indicating that the transfer of AchrDNAs through *Agrobacterium* happens in rice as well (Ülker et al., 2008).

The present invention is based on the surprising finding that several genetic elements can be held responsible for the vast majority of the undesired transfer events. These are short DNA regions (cis-elements) in *Agrobacterium* chromosomes which are responsible for transfer of flanking bacterial DNAs to plant genomes during plant transformation. Identified were a) the *OriT*-like (origin of transfer like) region found in the *HS1*_{LC} (hot spot 1 on *Agrobacterium* linear chromosome) which is responsible for the majority of the *A*chrDNA transfer from the linear *Agrobacterium* chromosome, and b) the *RB*-like (right border like) element found on the *HS1*_{CC} (hot spot 1 on *Agrobacterium* circular chromosome) and is responsible transferring an AchrDNA region in the circular chromosome. Thus, the presence of at least one of these elements constitutes a biosafety risk. Furthermore, IS426, a particularly active insertion sequence (transposon) which has two full length copies (IS426 copy I and IS426 copy II), one partial and circular transposition intermediate copy, has been identified. The transposon can jump into the T-DNA regions in plant transformation vectors and with T-DNA is transferred to plants. As was furthermore shown, IS426 can also mutate or activate genes (especially antibiotic resistance genes) in bacteria. Therefore, IS426 is also a particular biosafety risk factor.

The present inventors now showed that the problematic genetic elements as identified can be removed (e.g. deleted) from *Agrobacterium* genome without a negative effect on the viability or effectiveness of the bacteria, as the deletion thereof does not influence normal and desired T-DNA transformation processes. The strains as produced still contain the remnants of antibiotic resistance cassettes as introduced to aid selection of homologous recombination mediated deletion events. Nevertheless, such antibiotic resistance genes can readily be removed from the strain by the person of skill in order not to limit the number of selectable marker genes that can be used when engineering this bacterium further. The following strains were constructed and tested.

The desired deletions can be introduced into the *Agrobacterium tumefaciens* strains according to the invention by using any method known to the person of skill, such as, for example, using suicide vectors comprising suitable resistance markers, such as, for example, antibiotic resistance markers, such as kanamycin resistance.
- An IS*426* copy I deletion strain in an *Agrobacterium tumefaciens* A136 background;
- An IS426 copy II deletion strain, also in an *Agrobacterium tumefaciens* A136 background;
- An IS*426* copy I and IS*426* copy II deletion strain in an *Agrobacterium tumefaciens* A136 background; and
- An *OriT* deletion strains in an *Agrobacterium tumefaciens* GV3101 pMP90 background.

Encompassed by the present invention is an *RB*-like element deletion strain, which can be generated in *Agrobacterium tumefaciens* in analogy to the above strains. Further included are an IS*426* copy I, IS*426* copy II deletion, and *OriT* deletion strain in an *Agrobacterium tumefaciens* background; and an IS*426* copy I and *OriT* deletion strain in an *Agrobacterium tumefaciens* background; and an IS*426* copy II deletion and *OriT* deletion strain in an *Agrobacterium tumefaciens* background.

In a particularly preferred embodiment of the present invention, the invention relates to an IS*426* copy I, IS*426* copy II deletion, *RB*-like element and *OriT* element deletion strain in an *Agrobacterium tumefaciens* background. It is expected that this strain will be nearly devoid of transforming or transferring undesired sequences into the cell to be transformed. The term "deletion strain" also encompasses strains carrying functionally inactivating mutations.

In a particularly preferred embodiment of the present invention, a desired bacterium has the genotype of deletions of two full length and active insertion sequences, IS*426* copy I and IS*426* copy II from the linear chromosome, a deletion of 61 bp *OriT*-like in the *HS1*_{LC} region on the linear chromosome, and, a deletion of 30 bp *RB*-like sequence in the *HS1*_{CC} on the circular chromosome, as described also below.

In yet another aspect of the present invention, the invention relates to an *Agrobacterium tumefaciens* strain according to the present invention, further comprising a recombinant chromosomally integrated minimal TypeIV secretion system (TypeIV SS), optionally comprising *virD2.* This will simplify plant transformation because there will be no more need for the use of a binary vector system, and thus helper plasmids. The bacteria will grow faster, and the use of antibiotic resistance genes is minimal, which will allow a better use of the markers in molecular biology work involving Agrobacterium as a host.

In yet another aspect of the present invention, the invention then relates to a method for producing an *Agrobacterium tumefaciens* strain according to the present invention, comprising the step of introducing at least one deletion in a sequence selected from the group of IS426 copy I, IS426 copy II, the *OriT*-like sequence, and the *RB*-like sequence in said strain.

Preferred is a method for producing an *Agrobacterium tumefaciens* strain, additionally comprising introducing a recombinant chromosomally integrated minimal TypeIV secretion system (TypeIV SS), optionally comprising *virD2,* into an *Agrobacterium tumefaciens* strain according to the present invention.

In yet another aspect of the present invention, the invention then relates to a method for transforming a cell selected from the group consisting of a plant, yeast, fungal, and human cell with a recombinant nucleic acid, comprising contacting said cell with an *Agrobacterium tumefaciens* strain according to the present invention, wherein said strain carries said recombinant nucleic acid to be transformed. Respective methods for *Agrobacterium tumefaciens* transformation are very well known in the art, and can be readily adapted by the person of skill. The present *Agrobacterium tumefaciens* strains do not require substantially different transformation conditions, compared to a non-modified *Agrobacterium tumefaciens* strain.

The present invention provides a number of important improvements for the field of cellular (In particular plant) transformation using the *Agrobacterium* T-DNA transformation system (also known as *Agrobacterium*-mediated (plant) transformation). The system and the strains of the present invention can be used to generate transgenic crops, to analyze the role of chromosomal DNA transfer in bacteria host interactions and disease development, for the transformation of yeast and fungi, and even for the transformation of animal and human cells. The system can be used to deliver proteins/genes designed or produced in *Agrobacterium* into these cells as well.

The present invention will now be further explained in the following examples with reference to the accompanying figures, nevertheless, without being limited thereto. For the purposes of the present invention all references as cited herein are incorporated by reference in their entireties. In the Figures,
Figure 1 shows that in addition to the T-DNA, Agrobacterium transfers very large fragments of its chromosomal DNA (*A*chrDNA) into plants.
Figure 2 shows acronyms and labels used to describe genotypes of Agrobacterium strains as generated and used in this patent application.
Figure 3 shows the strains used to determine mechanisms of bacterial chromosomal DNA transfer.
Figure 4 shows the promoter trapping assay used in the determination of bacterial chromosomal DNA transfer.
Figure 5 schematically shows an active insertion sequence in *Agrobacterium tumefaciens* genome that was identified using the promoter trapping assay.
Figure 6 shows a schematic depiction of the structure and putative coding sequences within *IS*426.
Figure 7 shows a schematic depiction of the PCR analysis and sequencing of the PCR products that led to the detection of IS*426* circles (possible transposition intermediates).
Figure 8 shows the results of the southern blot analysis to determine IS*426* copy numbers in selected *Agrobacterium* strains.
Figure 9 shows the generation of IS*426* deletion strains of *Agrobacterium.* The Southern blot analysis to determine IS*426* copy numbers in engineered, non-virulent, cured *Agrobacterium* strain A136 is shown (right side) in order to indicate indeed that both full copies are deleted.
Figure 10 shows a schematic depiction of *Agrobacterium* chromosomal DNA hot spots that were labelled by inserting a GFP expression cassette (active only in plants).
Figure 11 shows that *HSI*_{LC} tagged with GFP shows that *Agrobacteria* transfer the tagged region into plants.
Figure 12 shows the results of tagging other hot spots and non-hot spots with *GFP* in GV3101 pMP90 *Agrobacteriun* strain and plant transformation using the transient assay.
Figure 13 shows that tagging *HS1*_{LC} with *GFP* in selected Agrobacterium strains identified that chromosomal DNA is VirD2 and TypeIV SS dependent.
Figure 14 shows the map of pBasicS1-GFP vector used in testing cis elements involved in chromosomal DNA transfer.
Figure 15 shows the results of the searches for DNA regions at or around the hot spots that led to the discovery of *OriT*-like and *RB*-like sequences responsible for *A*chrDNA transfer.
Figure 16 shows that A) the predicted *OriT*-like sequence in the linear chromosome hot spot 1 is responsible for transferring this region from bacteria to plants, and B) that the predicted *RB-*like sequence in the circular chromosome hot spot 1 is responsible for transferring this region from bacteria to plants.

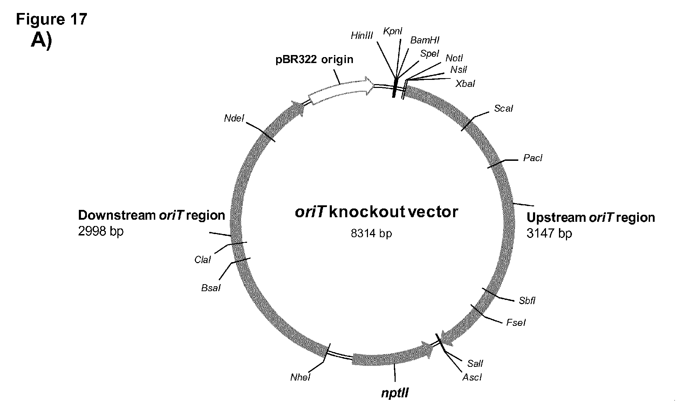

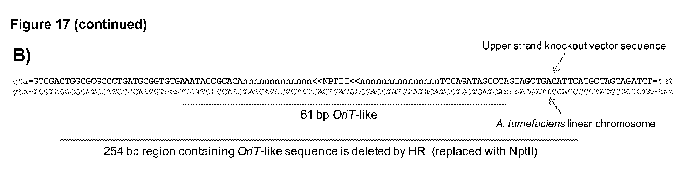

Figure 17 shows the strategy used in deletion of the *OriT*-like sequences is depicted. (A) The recombination vector is a suicide plasmid and cannot replicate in Agrobacterium. It contains bacterial expression cassette for the kanamycin resistance gene *nptII* flanked by the Agrobacterium sequences determining the position of recombination mediated deletion of sequences from bacterial genome but addition of *nptII* expression cassette. (B) Transformation of the recombination vector into *HS1*_{LC} *GFP*-tagged GV3101 pMP90 Agrobacterium cells and selection of bacteria by kanamycin resulted in double recombination mediated replacement of *nptII* with the *OriT*-like sequence on the linear chromosome
Figure 18 shows that the deletion of *OriT*-like sequence from the linear chromosome hot spot 1 stopped also majority of chromosomal DNA transfer from linear chromosome hot spot 2 indicating that their transfer are linked and OriT-like sequence is responsible transfer of both regions to plants.
Figure 19 shows schematic drawings of the genotypes of preferred BioSAFE *Agrobacterium* strains and associated vector systems according to the present invention.
Figure 20 shows an alignment identifying the left border, right border, and the border-like sequence according to the present invention based on alignments with RB or LB. LB, non italics; RB, italics; Nucleotides aligning neither LB nor RB are in small case, aligning sequences are in capital letters and are underlined.
Figure 21 shows that the predicted *OriT*-like sequence in the linear chromosome hot spot 1 is responsible for transferring this region from bacteria to plants, similar to Figure 16A.

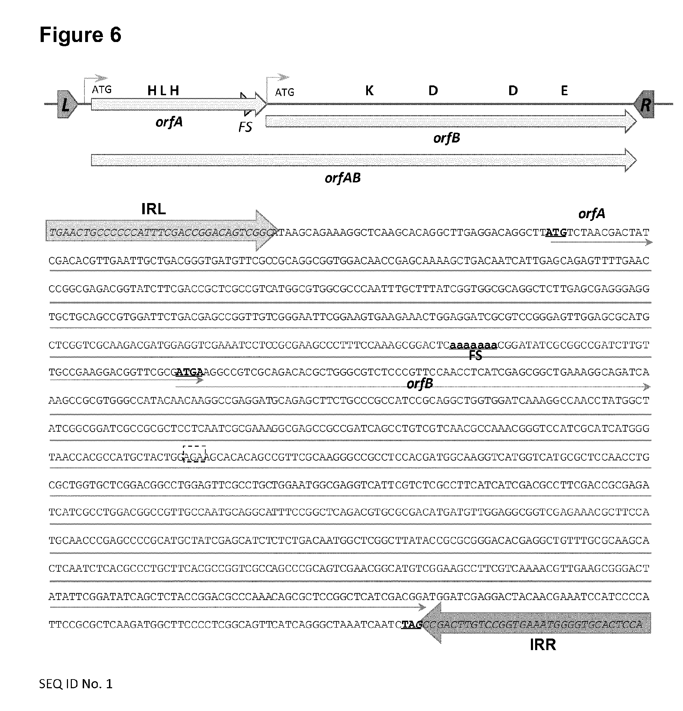

SEQ ID No. 1 to 3 show the sequences of IS426 copy I, II, and III, respectively. (see Figure 6)

SEQ ID No. 4 to 6 show the amino acid sequences of ORFA, ORFB, and ORFAB, respectively. (see Figure 6)

SEQ ID No. 7 shows the nucleotide sequence of the oriT region (61 bp). (see Figure 17)

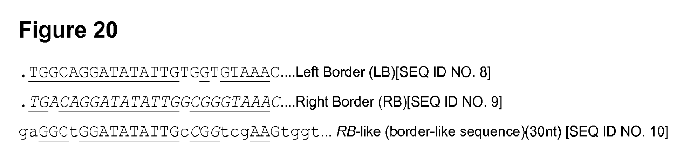

SEQ ID No. 8 and 9 show the right and left border sequences, respectively, and SEQ ID No. 10 shows the border-like sequence according to the invention. (see Figure 20)

### EXAMPLES

In Europe and most of the world, *Agrobacterium tumefaciens* is classified under the risk group 1, therefore it can be used in research and development in all lowest security level (S1) laboratories. There are various *Agrobacterium* strains developed by researchers throughout the world. Recently, such strains are becoming commercially available (e.g. from Takara Bio, JP).

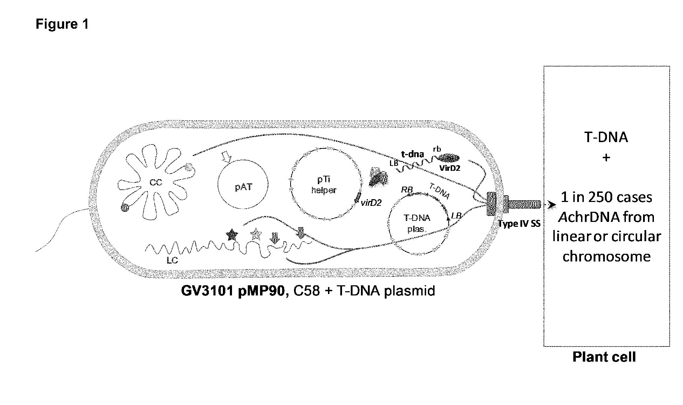

However, as the inventors have demonstrated in 2008, the available *Agrobacterium* strains have a hidden biosecurity risks. These bacteria appear to transfer very large fragments of its chromosomal DNA (*A*chrDNA) besides the DNA of interest which is typically cloned within the transferred region (T-DNA) whose limits are defined by 25 bp direct repeats which are termed "right and left border" (Ülker et al., 2008) (see Figure 1).

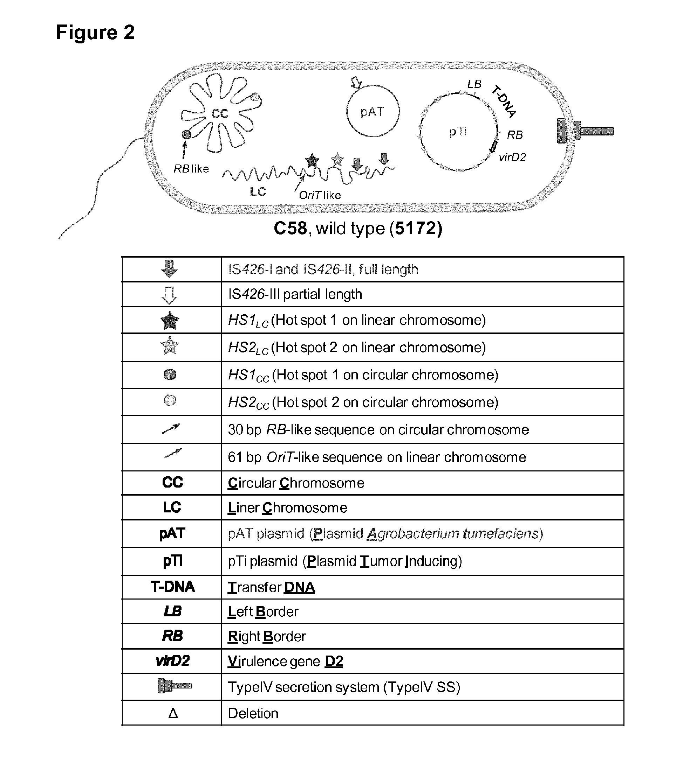

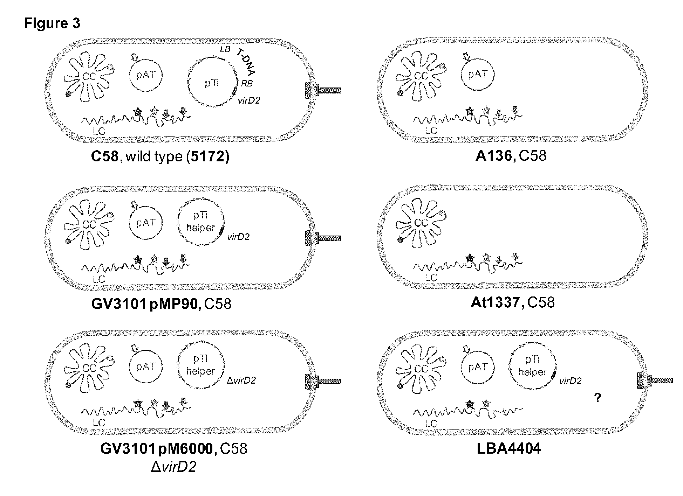

Since many regions and plasmids are manipulated in the examples, Figure 2 provides a simple diagram with acronyms and shapes are given for better comprehension of the work that has been done (see Figure 2). Similarly, since many different strains of Agrobacterium with different genotypes are used, simple diagrams showing their characteristics are given in Figure 3.

### 1. Mechanisms of AchrDNA transfer: Transposon IS426

### Promoter trapping resulted in mostly IS426 transposition into T-DNA vector

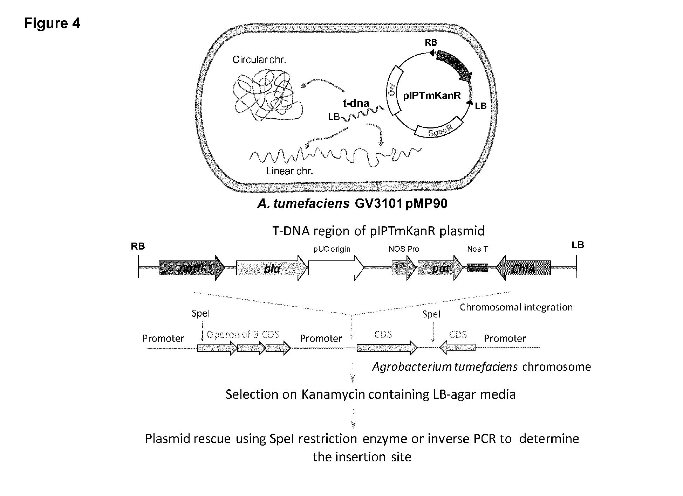

In order to determine, how *Agrobacterium* chromosomal DNA fragments (*A*chrDNAs) other than T-DNAs are unintentionally transferred from the bacteria to plants, the inventors tested various possible mechanisms. Integration of T-DNA into bacterium's own chromosomes and a re-launch from the chromosomes together with some flanking *A*chrDNAs and their subsequent transfer to plants was one of the theories (Ülker et al., 2008). To test this theory, a trapping method which was expected to report insertion of T-DNA into Agrobacterium chromosomes was designed. The inventors called this method "insertional promoter trapping mediated kanamycin resistance" (IPTmKanR). The strategy relies on trapping promoters using a promoterless kanamycin resistance gene located at the right border of a T-DNA plasmid by growing bacteria on kanamycin containing LB plates (Figure 4). Insertion of T-DNAs in various locations in bacterial chromosomes was expected as occasional insertions next to a promoter which could be sufficient to drive expression of the resistance gene and apperance of kanamycin resistant colonies.

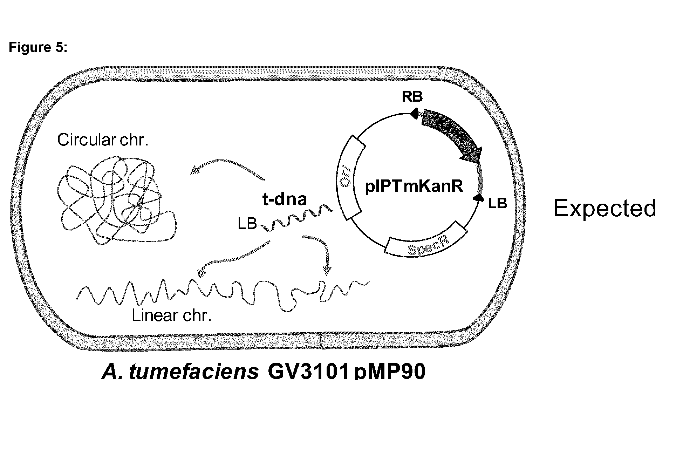

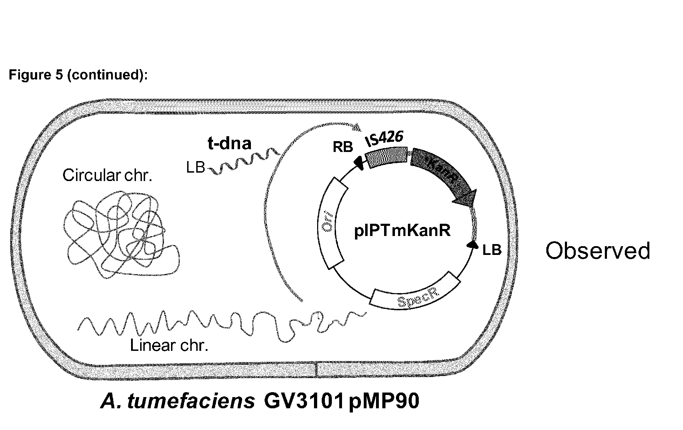

The inventors obtained several kanamycin resistant Agrobacterium colonies. When the incubation time of plates at 28°C was increased, the number of colonies resistant to kanamycin was also increased. Incubation of bacteria for five days on kanamycin selection plates resulted in between 40 to 80 colonies. Agrobacteria carrying no T-DNA plasmid or an unrelated plasmid without kanamycin resistance gene gave also 5-10 colonies, indicating that Agrobacterium has an alternative kanamycin resistance mechanism. The inventors picked more than 50 colonies appearing at different times on kanamycin plates. Interestingly, analysis of these colonies indicated that instead of trapping chromosomally integrated T-DNAs, mostly (~61%) those cases were recovered, where an insertion sequence, IS426 copy from the *Agrobacterium* chromosomes was transposed upstream of the kanamycin resistance gene in the binary plasmid (Figure 5). The rest of the resistant colonies were due to either rearrangements in plasmid or intrinsic resistance mechanisms present in the bacteria.

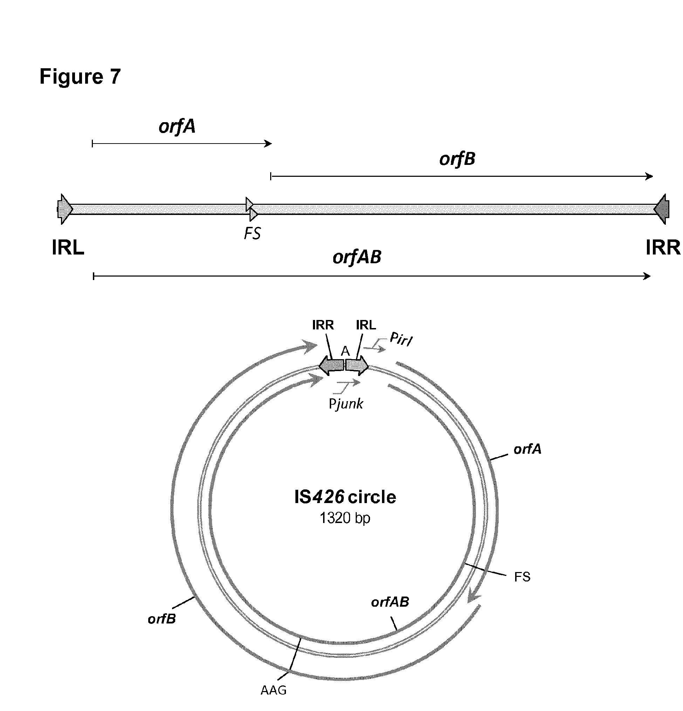

IS*426* was first described in the literature in 1986, and was designated IS*136* (Vanderleyden et al., 1986). Later, this name was changed to IS*426*. There were no other studies on this IS element. The study of Vanderleyden et al was short and did not contain detailed information. The authors reported that this IS element leads to a 9 bp duplication at the insertion site. However, later it was found that it leads to 5 bp duplications. A second publication appeared in 1999, and reported that the insertion of IS*426* was responsible for disruption of tetracycline resistance in *Agrobacterium* (Luo and Farrand, 1999). Lately, other publications reporting the presence of IS*426* in T-DNA plasmids were also published (Llop et al. 2009; Rawat et al., 2009), however none of these studies were directed at the characterization or removal of IS*426* from the Agrobacterium genome. Figures 6 and 7 give some key features of this element relevant to biosafety.

### IS426 copy numbers and transposition mechanisms

Bioinformatics analysis showed the presence of two full-length and one partial copy of the *IS*426 in the sequenced *A. tumefaciens* C58 genome. The partial copy is located on the pTA plasmid, but both full-length copies are located on the linear chromosome. The full-length copies can be distinguished, because one of them has a three nucleotide (or one amino acid) deletion in the orfB region (Figure 6). Bioinformatics analysis also suggested that IS*426* has two putative, non-overlapping open reading frames (ORFs) (Figure 6). The inventors furthermore detected a Chi sequence (AAAAAAA) between orfA and orfB. Such stretches of A's are shown to cause frame shifting during translation by ribosomes in other transposon coding sequences. Indeed, frame shifting in these A's of one nucleotide forward leads to orfAB which is different from both orfA and orfB.

Figure 6 shows the structure and putative coding sequences within *IS*426; there are two full length and one partial copy of the IS*426* in the sequenced *A. tumefaciens* C58 genome. The full length IS*426* I is 1319 bp and IS*426* II is 1316 long (missing 3 nucleotides are highlighted by gray) and both are located on the linear chromosome. The partial copy is called IS*426* III, and located on the pTA plasmid.

During PCR analysis with inverse primers specific for IS*426*, the inventors have also identified plasmid like episomal circles of IS*426* in *Agrobacterium* cells (Figure 7). These are possible transposition intermediates.

### Transposition mechanism of IS426

To determine the transposition strategy of the *IS*426 element, the inventors developed a simple method. If the transposition is carried out by a cut and paste mechanism, the IS element should no longer be detected in the original sequenced location, however, if it is transposed as copy and paste mechanism, the IS element should retain its original location. Using analysis of the genomic DNA of IPTmKanR clones, where IS*426* copies are transposed into this vector and integrated upstream of the *nptII* gene, the inventors found that the other copies of the IS*426* are still in their original locations. This indicates that the mechanism of transposition functions not through cut and paste, but through copy and paste mechanism.

### Analysis of the IS426 copy number in the most frequently used Agrobacterium strains

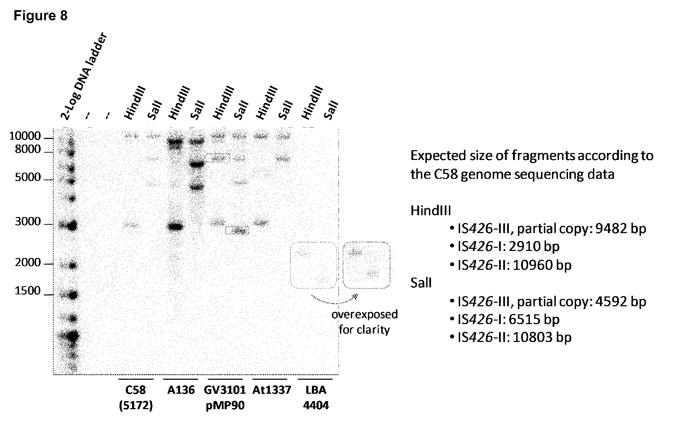

High frequency of transposition into a plasmid upon antibiotic stress indicated that **IS*426*** is an active transposon, and thus its copy number could have been different from the sequenced *A. tumefaciens* C58 strain. Therefore, the inventors performed DNA blot analysis with DNA isolated from *A. tumefaciens* C58 strain as well as several other important strains used in plant transformation. They combined DNA blot analysis data with inverse PCR and sequencing of the PCR fragments in order to determine the exact location of the IS*426* copies. All three copies of the **IS*426*** were found to be in the original location of the sequenced *A. tumefaciens* C58 strain (Figure 8). This indicates that the copy number and the position of IS*426* may be strictly controlled. A136 strain, a derivative of *A. tumefaciens* C58 lacking pTi plasmid carried also the same number of IS*426* as the C58 strain, indicating that none of the **IS*426*** copies is located on pTi plasmid.

Surprisingly, however, on DNA blot analysis the inventors detected an additional copy of **IS*426*** in the engineered *A. tumefaciens* strain GV3101 pMP90. Rescuing the additional copy from the genomic DNA of this strain showed that this fourth copy of IS*426* is located on the engineered Ti plasmid pMP90. The copy is inserted just upstream of *virK* gene whose function is still unknown (Hattori et al., 2001; Wilms et al., 2012). Analysis of LBA4404, an octopine type strain, by DNA blot analysis showed that this strain had either only one copy divergent from IS*426*, or a partial copy as indicated by a weak signal in the blot compared to other nopaline type strains.

### Deletion of IS426 copies using homologous recombination

After demonstration that IS*426* can transpose into plasmids and may cause disruption of genes (*virK, tetA,* transgenes within T-DNA are some examples), activation of genes (*nptII* example), or unintentional transfer to plants by transposing into T-DNA regions of binary plant transformation vectors, it was desirable to completely remove this active **IS*426*** element from the genome of the most frequently used *Agrobacterium* strains. This task was challenging, since there are two full and one partial copies of the **IS*426*** as well as readily detected episomal IS*426* circles. Furthermore, it was also possible that there would be a selective pressure for keeping these copies in their original location in the nopaline strains, and that removal of IS426 from these locations may cause adverse effects or may even be detrimental for the *Agrobacterium* strain.

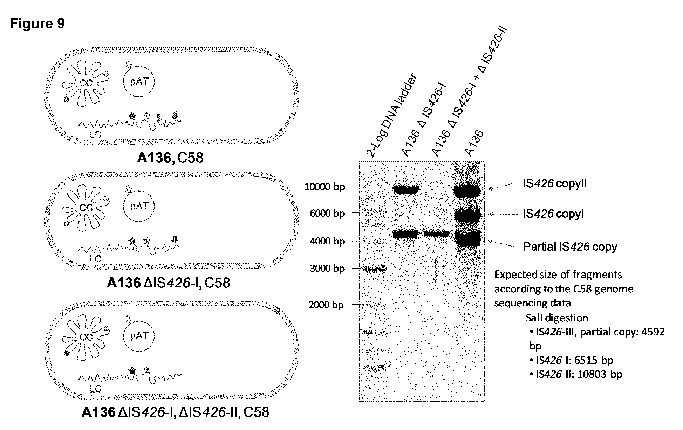

In order to stepwise remove the active **IS*426*** copies in the A136 model strain as used, the inventors generated homologous recombination vectors. These vectors, besides an antibiotic resistance gene, contained about 300 bp to 3000 bp flanking regions of the respective **IS*426*** copies. Furthermore, the vectors lacked origin of replication regions for plasmid maintenance in *Agrobacterium* (suicide vector). Upon transformation into *Agrobacterium* and selection with appropriate antibiotics, it was expected that the antibiotic resistance gene in this suicide vectors recombines with the respective IS426 copy through homologous regions flanking the antibiotic resistance gene, and thus leads to a replacement of IS*426* copy with the antibiotic resistance gene. Homologous recombination vectors with short homologies (300 to 400 bp) failed to delete the IS elements, however vectors having long homology stretches (1500 to 3000 bp) worked well and allowed the inventors to stepwise remove IS*426*-I and IS*426*-II. DNA blot analysis indicated that indeed these IS elements were indeed deleted from the A136 genome (Figure 9). Figure 9 shows the generation of IS*426* deletion strains of Agrobacterium. Southern analysis to determine IS*426* copy numbers in engineered, non-virulent, cured Agrobacterium strain A136 shows that both full copies are deleted.

### 2. MECHANISMS OF AchrDNA TRANSFER: OriT-like and RB-like sequences

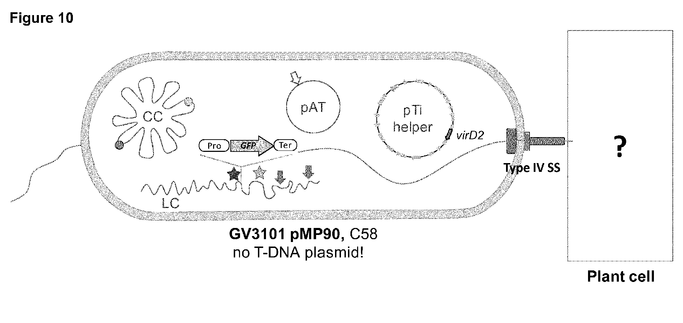

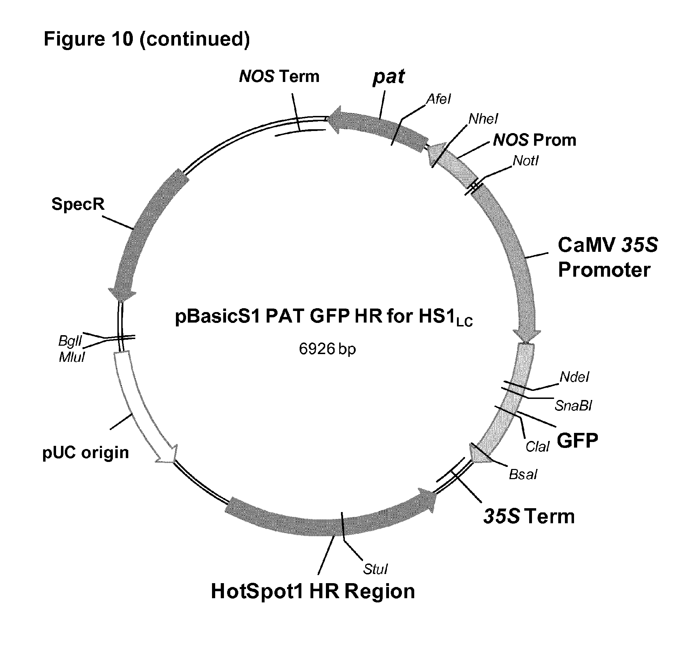

In order to determine the mechanisms of *Agrobacterium* chromosomal DNA (*A*chrDNA) transfer other than the IS*426* element from *Agrobacterium* to plants the inventors developed a test system to rapidly determine the transfer of other *A*chrDNAs to plants. A planta expression cassette for green fluorescent protein (GFP) (containing 35S promoter and NOS terminator) was introduced into selected hot spots using homologous recombination with a suicide plasmid conferring spectinomycin or kanamycin resistance-genes (Figure 10). The expected *GFP* tagging was confirmed by DNA blot (Southern) analyses in the engineered strains. *Nicotiana benthamiana* leaves were infiltrated with the engineered Agrobacterium strains, and GFP expression was determined three days post infiltration using a fluorescence microscope.

Figure 10 shows that *Agrobacterium* chromosomal DNA hot spots could be labelled one by one by inserting a GFP expression cassette (active only in plants). The *Agrobacterium* strains generated were used in transiently transforming tobacco leaves. Expression of GFP in plant leaves was indicative of a T-DNA independent mechanism of *A*chrDNA transfer. This assay was then used in order to determine the biological and genetic conditions that are required to eliminate the transfer.

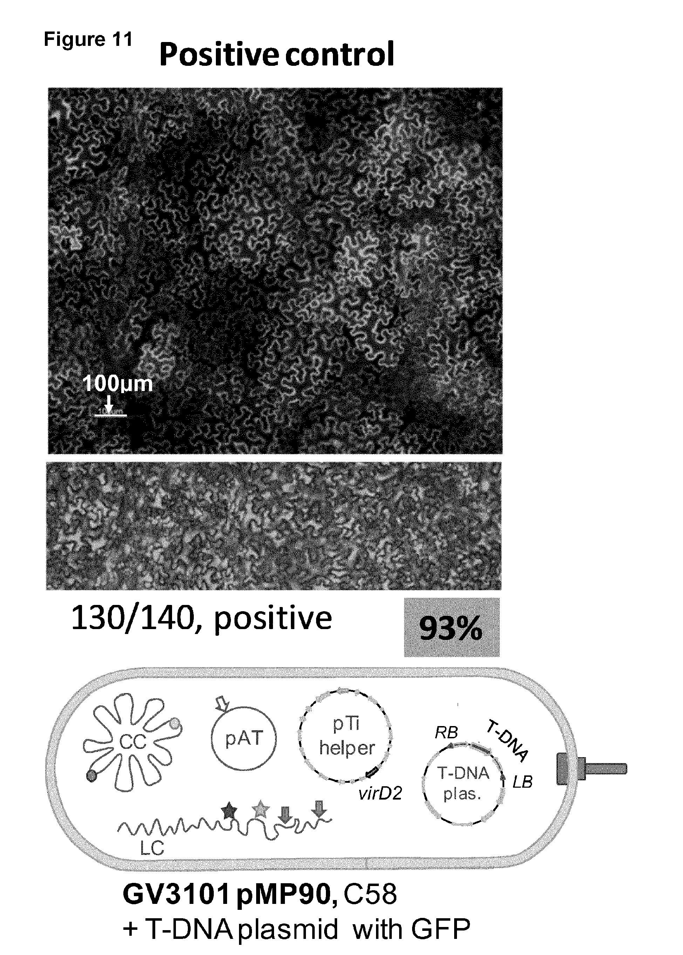

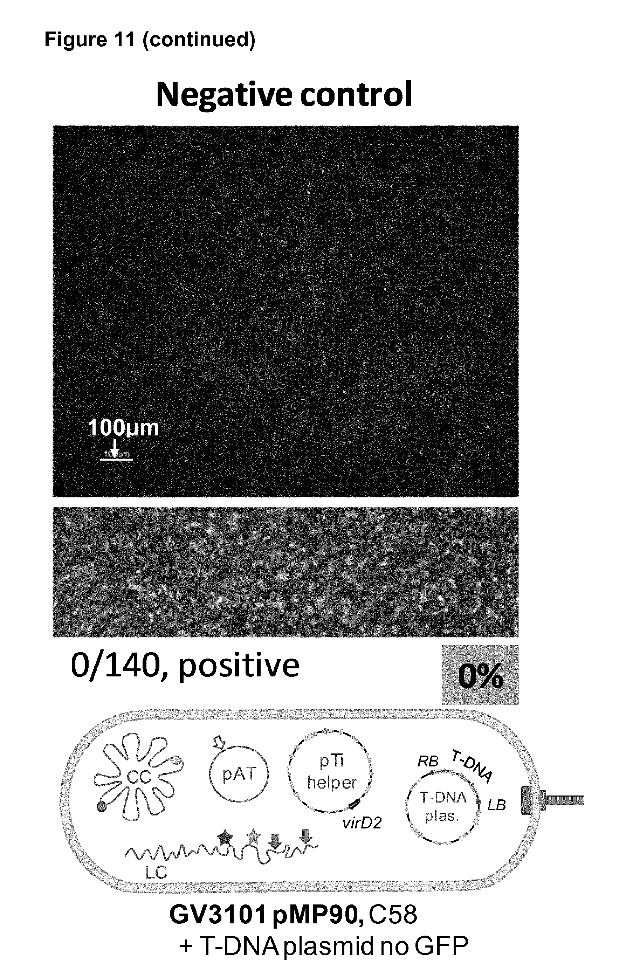

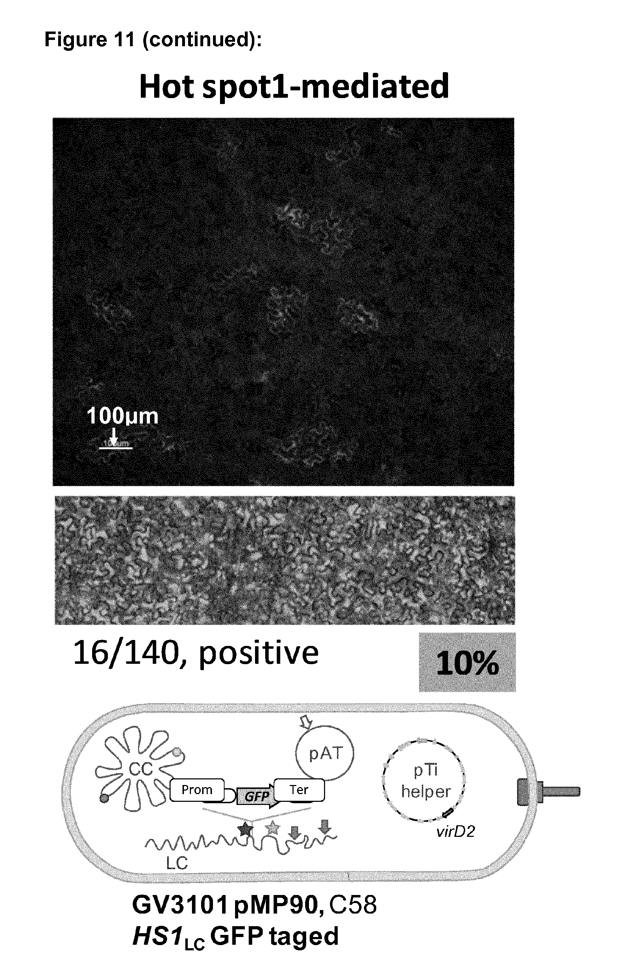

The tagging of the most frequently transferred hot spot on the linear chromosome of *Agrobacterium* (*HS1*_{LC}) with GFP in the GV3101 pMP90 strain and subsequent plant transformation assay showed that indeed such hot spots are transferred from bacterial chromosomes to plants (Figure 11). In Figure 11, the results of the homologous recombination mediated insertion of GFP tagging vector into the *A. tumefaciens* linear chromosome are shown. The recombination vector used is a suicide plasmid and cannot replicate in *Agrobacterium.* It contained the bacterial expression cassette for the spectinomycin resistance gene *aad41.* A plant expression optimized GFP expression cassette flanked by the *Agrobacterium* sequences determined the position of the recombination mediated insertion into bacterial genome. The *HS1*_{LC} *GFP*-tagged *Agrobacterium* strains in the background of GV3101 pMP90 *Agrobacterium* cells were then used for the transient transformation of tobacco leaves to determine, whether these regions in the *Agrobacterium* genome are transferred to plants.

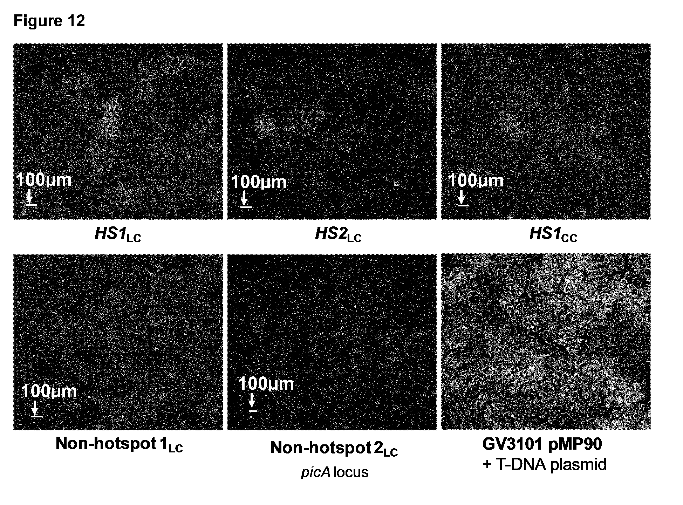

In addition and similar to *HS1*_{LC} tagging as above, also the second most frequently transferred hot spot on the linear chromosome of *Agrobacterium* (*HS2*_{LC}) was tagged with *GFP* in the GV3101 pMP90 strain. The subsequent plant transformation assay showed that this hot spot is transferred from bacterial chromosomes into plants (Figure 12).

Then, the tagging unrelated non-hot spots in *Agrobacterium* chromosome with *GFP* gave no GFP expression in plants, which shows that DNA transfer is indeed site specific, as shown in Figure 12, where tagging other hot spots and non-hot spots with *GFP* in GV3101 pMP90 Agrobacterium strain and plant transformation using transient assay is shown.

### AchrDNA transfer is VirD2 and TypeIV SS dependent

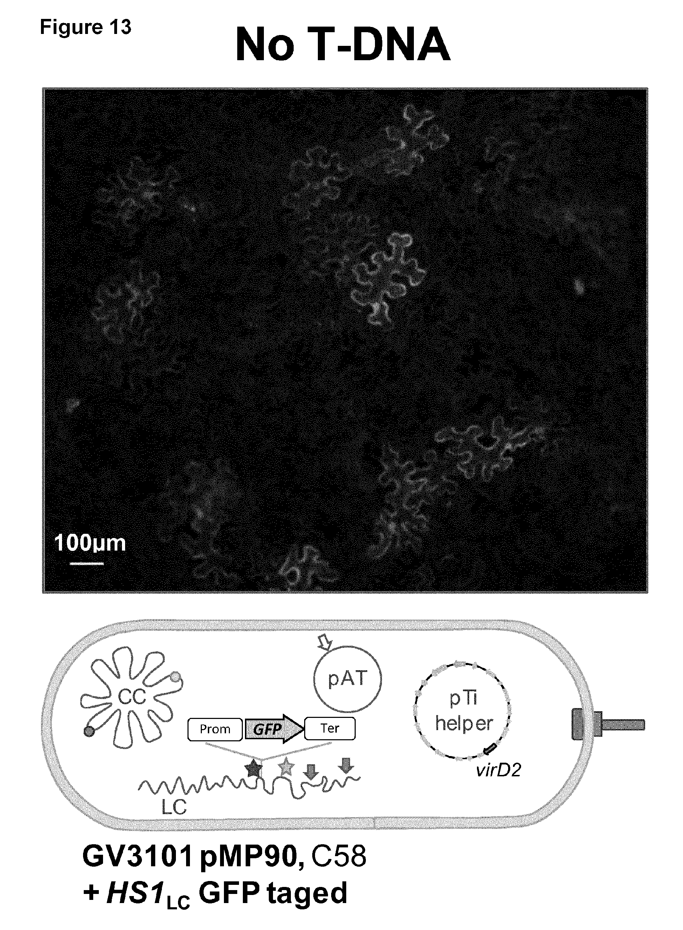

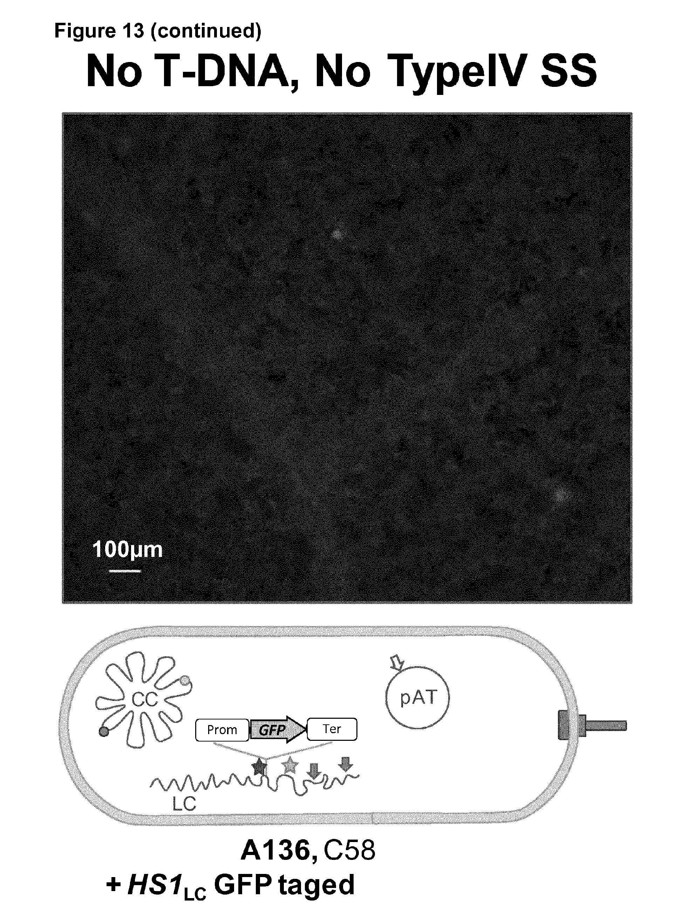

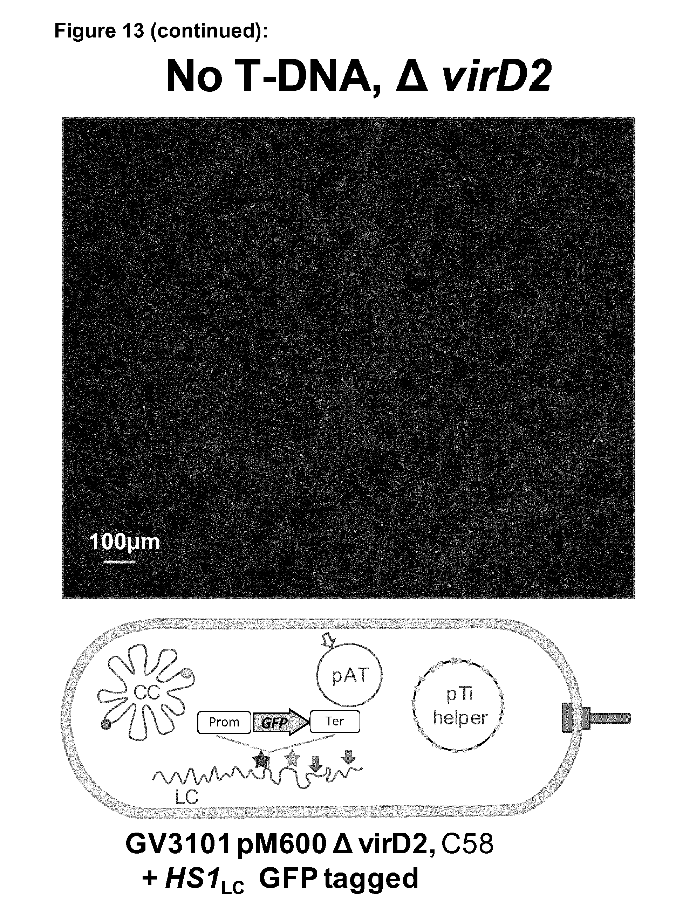

In addition to the T-DNA transfer system, *Agrobacterium* also contains many genes and secretion channels for conjugations of its plasmids. To determine how the DNA around hot spots are cleaved and transferred to plants, the inventors tagged the same regions in different *Agrobacterium* strains. *Agrobacterium* strain A136 was cured of the pTi plasmid, hence it has no TypeIV secretion system (SS) forming the injection channel, and VirD2 which is crucial in T-DNA transfer. On the other hand, the GV3101 pM600 ΔvirD2 strain contained the helper plasmid containing the TypeIV SS, but had a deletion of the *virD2* gene. Thus, as shown in Figure 13, a transfer of *HS1*_{LC} was blocked in both strains. This clearly indicates that like T-DNA, the processing of hot spot DNAs and their transfer into plants is both VirD2 and TypeIV SS dependent. In Figure 13, the results of the tagging of *HS1*_{LC} with *GFP* in selected *Agrobacterium* strains show that the processing of chromosomal DNA is VirD2 and TypeIV SS dependent.

### VirD2 cleavage sites at or around hot spots

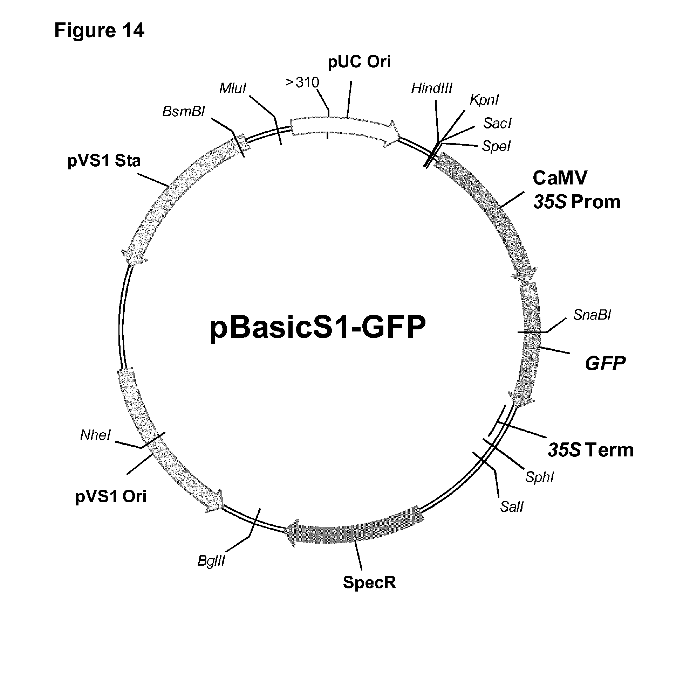

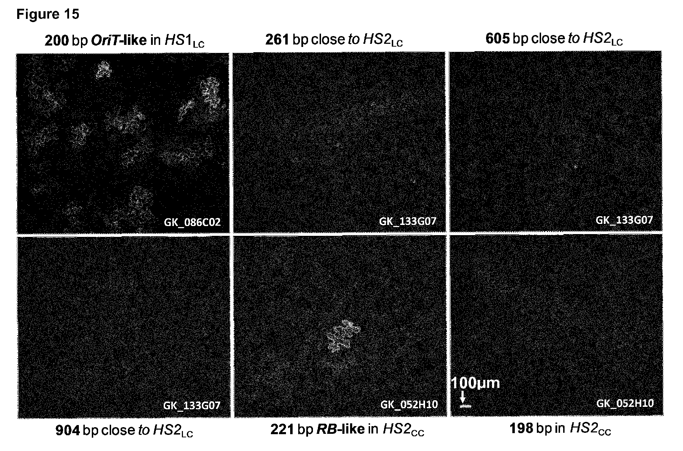

Once it was determined that AchrDNA transfer is VirD2 dependent, the inventors searched for T-DNA right and left borders (*RB* and *LB*) or *OriT* sequences which were also shown to be cleavable by VirD2 (Pansegrau et al., 1993). Nevertheless, the analysis resulted in no perfect matches to these sequences, and many mismatches (as low as 65% match) had to be allowed. With such a low similarity, the inventors identified several hundred matches scattered throughout all chromosomes. The analysis was narrowed to around the hot spots, and tests with various sizes of fragments were performed in order to determine VirD2 cleavage sites. For this, PCR amplified fragments from selected regions on Agrobacterium genome were clone into pBasicS1-GFP plasmid (Figure 14). pBasicS1-GFP, which can replicate in *Agrobacterium,* carries a GFP expression cassette that would report GFP expression in plants upon delivery. However, the plasmid had no T-DNA borders or origin of transfer sequences, therefore cannot transform plants with GFP. Fragments that were cloned into pBasicS1-GFP were transformed into GV3101 pMP90 *Agrobacterium* strain, and a transient plant transformation assay was carried out using *N. benthamiana* plants. As shown in Figure 15, the inventors identified two key fragments (200 bp *OriT*-like in *HS1*_{LC} and 221 bp *RB*-like in *HS2*_{CC}) that contained VirD2 cleavage sites. Figure 14 shows the map of the pBasicS1-GFP vector used in testing cis elements involved in chromosomal DNA transfer, and Figure 15 shows the results of the search for DNA regions at or around the hot spots for a discovery of *OriT*-like and *RB-*like sequences responsible for *A*chrDNA transfer. PCR amplified fragments from selected regions on *Agrobacterium* genome were cloned into the pBasicS1-GFP plasmid (has no T-DNA borders or origin of transfer sequences, therefore cannot transform plants with GFP). The plasmids were then transformed into the GV3101 pMP90 *Agrobacterium* strain, and a transient plant transformation assay was carried out. Images were taken three days after infiltration with bacteria OD₆₀₀=0.25. Many other fragments were tested, but showed no GFP expression in plants.

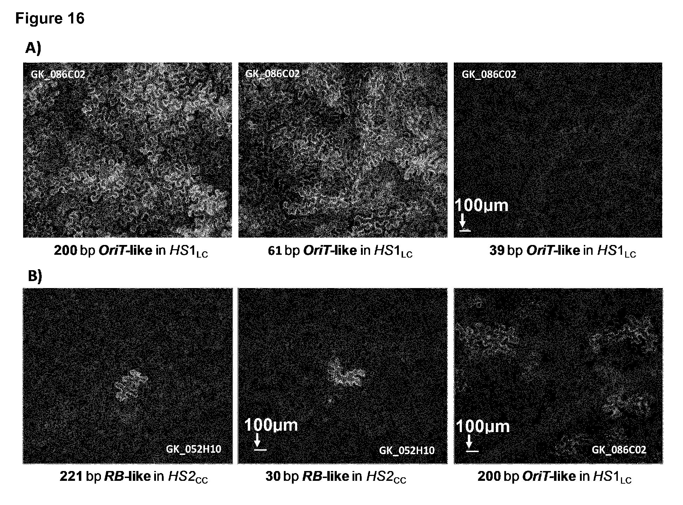

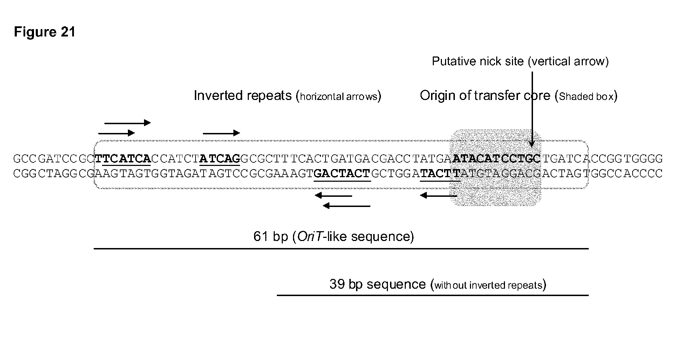

In order to prove that the *OriT*-like sequence and the *RB*-like sequence as present in the 200 and 221 bp fragments are actually cleavage sites for VirD2, the inventors then generated shorter fragments that only contained the core sequence (61 bp *OriT*-like and 30 bp *RB*-like). Figure 16A shows that - as expected - the predicted *OriT*-like sequence in the linear chromosome hot spot 1 is responsible for transferring this region from bacteria to plants. Origin of transfer sequences typically contain inverted repeats upstream of the core recognition sequence. Presence of these repeats is highly crucial for the functionality of the origin of transfer regions. As shown in Figure 21, the inventors have also found such short inverted repeats and their deletion from the predicted origin of transfer (39 bp sequence) also eliminated its function and hence transfers of *GFP* to plants (Figure 16A and 21). Core *OriT* region which is contained within the 39 bp sequence has a limited similarity to the left and right borders. Since this fragment was not functional without the upstream inverted repeats, the activity of this sequence is not border but *OriT.* Images were taken six days after infiltration with bacteria OD₆₀₀=0.3. Figure 16B shows that - as expected - the predicted *RB*-like sequence in the circular chromosome hot spot 1 is responsible for transferring this region from bacteria to plants. The 30 bp *RB*-like fragment was cloned into pBasicS1-GFP vector and tested in transient leaf transformation assays. Images were taken three days after infiltration with bacteria OD₆₀₀=0.3.

### Deletion of OriT-like from the genome of Agrobacterium tumefacies blocks the vast majority of chromosomal DNA transfer from HS1_{LC}

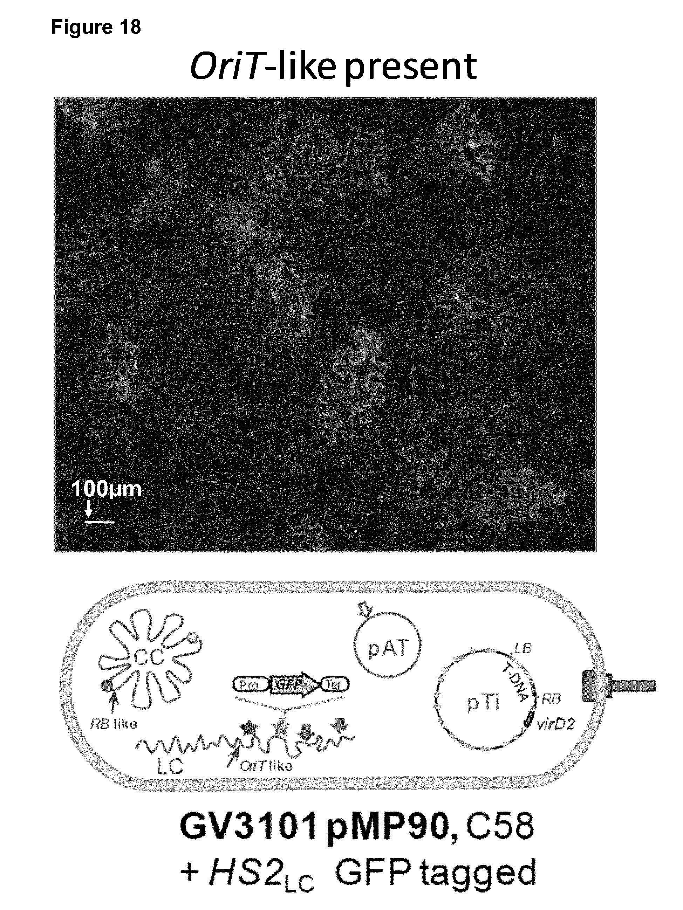

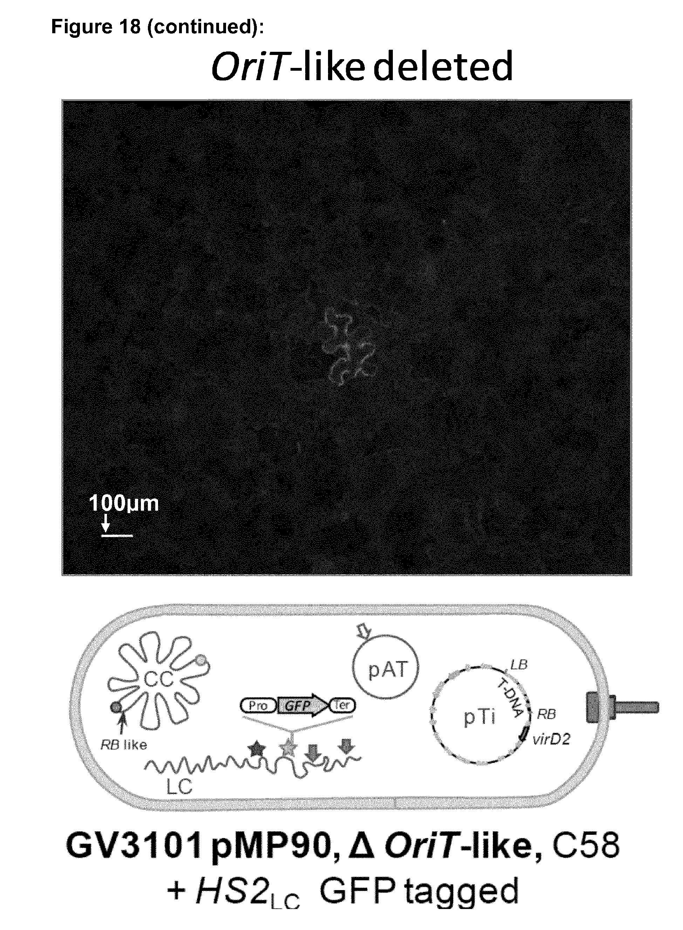

To further demonstrate that the elements as described above were responsible for chromosomal DNA transfer, the inventors first generated deletion mutants using homologous recombination for the *OriT*-like sequence on the linear chromosome. They used the *HS1*_{LC} *GFP* tagged GV3101 pMP90 Agrobacterium strain in order to knock out the *OriT*-like sequence (Figure 17). Deletion of this sequence from the genome of the strain blocked the vast majority of chromosomal DNA transfer into this locus (Figure 18). Some positive cells were still found, indicating that there may be at least one more sequence involved in a weak DNA transfer in or around this hot spot. Figure 17 shows the results of the homologous recombination mediated deletion of ***OriT*-like** sequence from the *A. tumefaciens* linear chromosome; and the strategy used in the deletion of the *OriT*-like sequences is depicted. (A) The recombination vector is a suicide plasmid and cannot replicate in *Agrobacterium.* It contains the bacterial expression cassette for the kanamycin resistance gene *nptII,* flanked by the *Agrobacterium* sequences determining the position of recombination mediated deletion of sequences from bacterial genome by the introduction of the *nptII* expression cassette. (B) Transformation of the recombination vector into *HS1*_{LC} *GFP*-tagged GV3101 pMP90 *Agrobacterium* cells and selection of bacteria by kanamycin resulted in double recombination mediated replacement of *nptII* with the *OriT*-like sequence on the linear chromosome. Figure 18 shows the results of the deletion of the *OriT*-like sequence from the linear chromosome hot spot 1, which stopped the vast majority of chromosomal DNA transfer from this hot spot. The homologous recombination-mediated deletion of *OriT*-like sequence was carried out in the GV3101 pMP90 strain, where hot spot 1 was tagged with the GFP expression cassette. Only very few positive plant cells were obtained comprising a deleted *OriT-*like sequence, suggesting that this sequence is the main source of chromosomal DNA transfer. Images were taken three days after infiltration with bacteria OD₆₀₀=0.3.

### HS1_{LC} and HS2_{LC} are linked, and the deletion of OriT-like from the genome of Agrobacterium tumefacies also blocks the vast majority of the chromosomal DNA transfer from HS2_{LC}

The second most frequently transferred hot spot on Agrobacterium chromosomes is *HS2*_{LC}*,* and this hot spot is located about 30 Kb downstream from *HS1*_{LC}*,* indicating that they may be linked. In order to determine, whether the transfer of these hot spots is linked, and whether the DNA transfer process is initiated at the ***OriT*-like** sequence, the inventors deleted the *OriT-*like sequence from *HS2_{LC} GFP* tagged GV3101 pMP90 *Agrobacterium* strain. Like in the case of *HS1*_{LC}, the transfer of *HS2*_{LC} into plants cells was mostly abolished, indicating that these hot spots are linked and DNA transfers are initiated at ***OriT*-like** sequence at *HS1*_{LC} (Figure 18). Furthermore, the inventors identified only very few positive cells, indicating again that there may be least one more weak sequence involved in DNA transfer in or around these hot spots. Figure 18 shows the results of the deletion of *OriT-*like sequence from the linear chromosome hot spot 1, which stopped also the vast majority of chromosomal DNA transfer from linear chromosome hot spot 2; indicating that their transfers are linked, and that the ***OriT*-like** sequence is responsible transfer of both regions to plants. The homologous recombination mediated deletion of *OriT*-like sequence was carried out in GV3101 pMP90 strain, where hot spot 2 was tagged with GFP expression cassette. Images were taken three days after infiltration with bacteria OD₆₀₀=0.3.

### Combination of the deletions in the genome of Agrobacterium tumefacies

A strain of Agrobacterium is constructed that combines the deletions of the *OriT-*like, *RB*-like and IS*426* copies as described above. This Agrobacterium strain shows only extremely low *A*chrDNA transfer to plants.

As a particular example, the AtC58-BioSAFE bacterium has the genotype of a deletion of the 61 bp *OriT*-like element in the *HS1*_{LC} region on the linear chromosome, a deletion of the 30 bp *RB*-like sequence in the *HS1*_{CC} region on the circular chromosome, and deletions of the two full length insertion sequences, IS*426* copy I and IS*426* copy II from the linear chromosome.

Furthermore, the strain will optionally contain the chromosomally integrated minimal Type IV secretion system (TypeIV SS). This will simplify plant transformation because there will be no more need for a binary system and helper plasmids. There are two alternatives, TypeIV SS containing *virD2* or not containing *virD2.* Transferring the core components of the TypeIV secretion system (TypeIV SS) from pTi plasmid into *Agrobacterium* linear chromosomes simplifies the so called binary (dual) vector system in plant transformation into a unitary (single component) system. In the binary system, the components of the DNA transfer machinery (tumor inducing plasmid, pTi plasmid) were divided into two plasmids (two components). The TypeIV SS (component one, also called the helper plasmid) forms the bacterial injection system as well as contains the key genes involved in processing and transferring T-DNA into plants. In the original pTi plasmid, there were genes causing tumor formation in plants within the T-DNA region. Therefore, this region is completely deleted from the helper plasmids. However, in order to transform plants with a desired DNA, a T-DNA vector where the 25 bp borders are present (but no longer the tumor causing genes) is necessary. Therefore, various T-DNA vectors (component two) were generated to aid researchers for cloning gene of interests within the T-DNA for plant transformation.

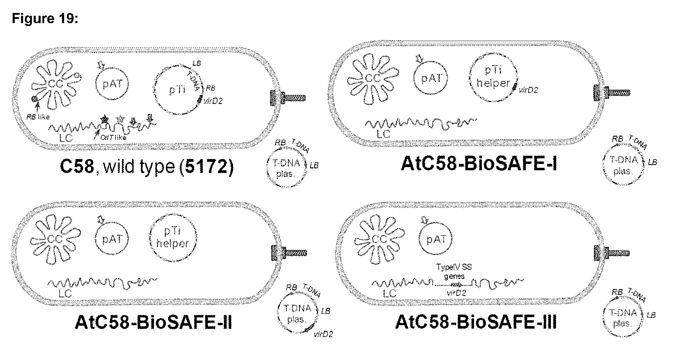

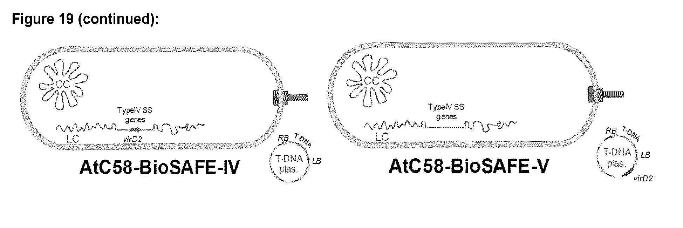

Figure 19 shows the desired BioSAFE Agrobacterium strains and associated vector systems. The AtC58-BioSAFE-I and II strains are in classical binary system except for the deleted sequences as described above. The *virD2* coding sequence from the helper plasmid is deleted to generate the AtC58-BioSAFE-II. The deleted *virD2* will be supplied again with the complementing plant transformation vector. The AtC58-BioSAFE-III, IV and V strains are in the unitary system as the minimal TypeIV SS genes necessary for channel formation and DNA/protein delivery to eukaryotic cells are inserted into the liner chromosome of the *Agrobacterium.* The AtC58-BioSAFE-III contains the pAT plasmid but the AtC58-BioSAFE-IV and V is devoid of it. The difference between AtC58-BioSAFE-IV and V is that virD2 is absent in the AtC58-BioSAFE-V genome, but will be supplied with the complementing plasmid vector.

### References as cited

Ülker, B., Li, Y., Rosso, M.G., Logemann, E., Somssich, I.E., and Weisshaar, B. (2008). T-DNA-mediated transfer of Agrobacterium tumefaciens chromosomal DNA into plants. Nat Biotechnol 26, 1015-1017.
Berson, T., Stirnberg, A. and Ülker, B. (2014). Characterization and elimination of IS426, an active insertion sequence of Agrobacterium tumefaciens*.* (in press)
Gelvin, S.B. (2008). Agrobacterium-mediated DNA transfer, and then some. Nat Biotechnol 26, 998-1000.
Grove, J.I, Alandiyjany, M.N., and Delahay, R.M. (2013). Site-specific Relaxase Activity of a VirD2-like Protein Encoded within the tfs4 Genomic Island of Helicobacter pylori. J Biol Chem 288: 26385-26396.
Hattori, Y., Iwata, K., Suzuki, K., Uraji, M., Ohta, N., Katoh, A., and Yoshida, K. (2001). Sequence characterization of the vir region of a nopaline type Ti plasmid, pTi-SAKURA. Genes Genet Syst 76: 121-130.
Llop, P., Murillo, J., Lastra, B., and Lopez, M.M. (2009). Recovery of nonpathogenic mutant bacteria from tumors caused by several Agrobacterium tumefaciens strains: a frequent event? Appl Environ Microbiol 75: 6504-6514.
Luo, Z.Q., and Farrand, S.K. (1999). Cloning and characterization of a tetracycline resistance determinant present in Agrobacterium tumefaciens C58. J Bacteriol 181: 618-626.
Pansegrau, W., Schoumacher, F., Hohn, B., and Lanka, E. (1993). Site-specific cleavage and joining of single-stranded DNA by VirD2 protein of Agrobacterium tumefaciens Ti plasmids: analogy to bacterial conjugation. Proc Natl Acad Sci U S A 90: 11538-11542.
Rawat, P., Kumar, S., Pental, D., and Burma, P.K. (2009). Inactivation of a transgene due to transposition of insertion sequence (IS136) of Agrobacterium tumefaciens. J Biosci 34: 199-202.
Vanderleyden, J., Desair, J., De Meirsman, C., Michiels, K., Van Gool, A.P., Chilton, M.D., and Jen, G.C. (1986). Nucleotide sequence of an insertion sequence (IS) element identified in the T-DNA region of a spontaneous variant of the Ti-plasmid pTiT37. Nucleic Acids Res 14: 6699-6709.
Wilms, I, Overloper, A., Nowrousian, M., Sharma, C.M., and Narberhaus, F. (2012). Deep sequencing uncovers numerous small RNAs on all four replicons of the plant pathogen Agrobacterium tumefaciens. RNA Biol 9: 446-457.

## Claims

1. *Agrobacterium tumefaciens* strain, comprising at least one deletion in a sequence selected from the group of IS*426* copy I, IS*426* copy II, the *OriT*-like sequence, and border-like sequences.

2. The *Agrobacterium tumefaciens* strain according to claim 1, wherein said strain comprises a deletion in two, three, or all four of said sequences.

3. The *Agrobacterium tumefaciens* strain according to claim 1 or 2, wherein the deletion of said sequence is partially or fully, such as, for example, 30 bp in the *RB*-like sequence, and/or 61 bp in the *OriT*-like sequence.

4. The *Agrobacterium tumefaciens* strain according to any one of claims 1 to 3, wherein said strain comprises a one or more nucleotide changes in one, two, three, or all four of said sequences.

5. The *Agrobacterium tumefaciens* strain according to any one of claims 1 to 4, wherein the *OriT-*like sequence is located in the *HS1*_{LC} region and the *RB*-like sequence is located in the *HS1*_{CC} region.

6. The *Agrobacterium tumefaciens* strain according to any one of claims 1 to 5, further comprising either a helper plasmid containing the TypeIV secretion system or a recombinant chromosomally integrated minimal TypeIV secretion system (TypeIV SS), optionally comprising *virD2.*

7. A method for producing an *Agrobacterium tumefaciens* strain according to any one of claims 1 to 6, comprising the step of introducing at least one deletion and/or inactivating /mutation in a sequence selected from the group of IS426 copy I, IS426 copy II, the OriT-like sequence, and the *RB*-like sequence in said strain.

8. A method for producing an *Agrobacterium tumefaciens* strain, comprising introducing a recombinant chromosomally integrated minimal TypeIV secretion system (TypeIV SS), optionally comprising *virD2,* into an *Agrobacterium tumefaciens* strain according to any one of claims 1 to 6.

9. A method for transforming a cell selected from the group consisting of a plant, yeast, fungal, and human cell with a recombinant nucleic acid, comprising contacting said cell with an *Agrobacterium tumefaciens* strain according to any one of claims 1 to 6 carrying said recombinant nucleic acid to be transformed.


SEQUENCE LISTING

<110>  Universität Bonn

<120>  Improved strains of Agrobacterium tumefaciens for transferring
       DNA into plants

<130>  R30485EP

<160>  10

<170>  PatentIn version 3.5

<210>  1
<211>  1319
<212>  DNA
<213>  Agrobacterium tumefaciens

<400>  1
tgaactgccc cccatttcga ccggacagtc ggcataagca gaaaggctca agcacaggct      60

tgaggacagg cttatgtcta acgactatcg acacgttgaa ttgctgacgg gtgatgttcg     120

ccgcaggcgg tggacaaccg agcaaaagct gacaatcatt gagcagagtt ttgaacccgg     180

cgagacggta tcttcgaccg ctcgccgtca tggcgtggcg cccaatttgc tttatcggtg     240

gcgcaggctc ttgagcgagg gaggtgctgc agccgtggat tctgacgagc cggttgtcgg     300

gaattcggaa gtgaagaaac tggaggatcg cgtccgggag ttggagcgca tgctcggtcg     360

caagacgatg gaggtcgaaa tcctccgcga agccctttcc aaagcggact caaaaaaacg     420

gatatcgcgg ccgatcttgt tgccgaagga cggttcgcga tgaaggccgt cgcagacacg     480

ctgggcgtct cccgttccaa cctcatcgag cggctgaaag gcagatcaaa gccgcgtggg     540

ccatacaaca aggccgagga tgcagagctt ctgcccgcca tccgcaggct ggtggatcaa     600

aggccaacct atggctatcg gcggatcgcc gcgctcctca atcgcgaaag gcgagccgcc     660

gatcagcctg tcgtcaacgc caaacgggtc catcgcatca tgggtaacca cgccatgcta     720

ctggagaagc acacagccgt tcgcaagggc cgcctccacg atggcaaggt catggtcatg     780

cgctccaacc tgcgctggtg ctcggacggc ctggagttcg cctgctggaa tggcgaggtc     840

attcgtctcg ccttcatcat cgacgccttc gaccgcgaga tcatcgcctg gacggccgtt     900

gccaatgcag gcatttccgg ctcagacgtg cgcgacatga tgttggaggc ggtcgagaaa     960

cgcttccatg caacccgagc cccgcatgct atcgagcatc tctctgacaa tggctcggct    1020

tataccgcgc gggacacgag gctgtttgcg caagcactca atctcacgcc ctgcttcacg    1080

ccggtcgcca gcccgcagtc gaacggcatg tcggaagcct tcgtcaaaac gttgaagcgg    1140

gactatattc ggatatcagc tctaccggac gcccaaacag cgctccggct catcgacgga    1200

tggatcgagg actacaacga aatccatccc cattccgcgc tcaagatggc ttcccctcgg    1260

cagttcatca gggctaaatc aatctagccg acttgtccgg tgaaatgggg tgcactcca     1319

<210>  2
<211>  1316
<212>  DNA
<213>  Agrobacterium tumefaciens

<400>  2
tgaactgccc cccatttcga ccggacagtc ggcataagca gaaaggctca agcacaggct      60

tgaggacagg cttatgtcta acgactatcg acacgttgaa ttgctgacgg gtgatgttcg     120

ccgcaggcgg tggacaaccg agcaaaagct gacaatcatt gagcagagtt ttgaacccgg     180

cgagacggta tcttcgaccg ctcgccgtca tggcgtggcg cccaatttgc tttatcggtg     240

gcgcaggctc ttgagcgagg gaggtgctgc agccgtggat tctgacgagc cggttgtcgg     300

gaattcggaa gtgaagaaac tggaggatcg cgtccgggag ttggagcgca tgctcggtcg     360

caagacgatg gaggtcgaaa tcctccgcga agccctttcc aaagcggact caaaaaaacg     420

gatatcgcgg ccgatcttgt tgccgaagga cggttcgcga tgaaggccgt cgcagacacg     480

ctgggcgtct cccgttccaa cctcatcgag cggctgaaag gcagatcaaa gccgcgtggg     540

ccatacaaca aggccgagga tgcagagctt ctgcccgcca tccgcaggct ggtggatcaa     600

aggccaacct atggctatcg gcggatcgcc gcgctcctca atcgcgaaag gcgagccgcc     660

gatcagcctg tcgtcaacgc caaacgggtc catcgcatca tgggtaacca cgccatgcta     720

ctggagcaca cagccgttcg caagggccgc ctccacgatg gcaaggtcat ggtcatgcgc     780

tccaacctgc gctggtgctc ggacggcctg gagttcgcct gctggaatgg cgaggtcatt     840

cgtctcgcct tcatcatcga cgccttcgac cgcgagatca tcgcctggac ggccgttgcc     900

aatgcaggca tttccggctc agacgtgcgc gacatgatgt tggaggcggt cgagaaacgc     960

ttccatgcaa cccgagcccc gcatgctatc gagcatctct ctgacaatgg ctcggcttat    1020

accgcgcggg acacgaggct gtttgcgcaa gcactcaatc tcacgccctg cttcacgccg    1080

gtcgccagcc cgcagtcgaa cggcatgtcg gaagccttcg tcaaaacgtt gaagcgggac    1140

tatattcgga tatcagctct accggacgcc caaacagcgc tccggctcat cgacggatgg    1200

atcgaggact acaacgaaat ccatccccat tccgcgctca agatggcttc ccctcggcag    1260

ttcatcaggg ctaaatcaat ctagccgact tgtccggtga aatggggtgc actcca        1316

<210>  3
<211>  955
<212>  DNA
<213>  Agrobacterium tumefaciens

<400>  3
gccccccatt tcgaccggac agtctgcata agcagaaagg ctcaagcaca ggcttggtta      60

taggcttatg tctaacgagt atcgacacgt tgaattgctg acgggtgatg ttcgccccag     120

gcggtggaca accgagcaaa agctgacaat cattgagcag agtttcttac ccggcgagac     180

ggtatcttcg accgctcgcc gtcatggcgt cgcgcccaat ttgctttatc ggtggcgcag 240

gctcttgagc gagggaggtg ctgcagccgt ggattctgac gagccggttg tcgggaattc 300

ggaagtgaag aaactggagg atcgcgtccg ggagttggag cgcatgctca gtcgcaagac 360

gatggaggtc gaatcctccg cgaagccctt accaaaaaaa cggatatcgc ggccgatctt 420

gttgccgaag gacggttcgc gatgaagacc gtcgcagaca cgctgggcgt ctcccgatcc 480

aacctcatcg agcggctgaa aggcaaatca aagccgcgtg ggccatacaa caaggtcgag 540

gatgcagaga ttttgcccat catccgcagg ctggtggatc aaatgccaac ctatggctat 600

cggcggatcg ccgcgctcct caatcgcgaa gggcgagccg ccgataagcc tgtcgtcaac 660

gctaaacggg ttcatcgcat tatgggcaac cacgcgatgc tgctcgagaa gcacacagcc 720

gttcgcaagg gccgcctcca cgacggcaag gtgatggtcg tgcgctcgaa cttgcgctgg 780

tgctcggatg ggctggagtt cacttgctgg aacggcgagg tcatccgtct cgccttcatc 840

atcgacgctt tcgactggga tatcatcgcc tggacggcgg ccgccaacgc gggcatctcc 900

ggctctgacg tgcgcgacat gatgctggag gcggtcgaaa agcggttcgc agcaa 955

<210> 4
<211> 129
<212> PRT
<213> Agrobacterium tumefaciens

<400> 4

Met Ser Asn Asp Tyr Arg His Val Glu Leu Leu Thr Gly Asp Val Arg
1               5                   10                  15

Arg Arg Arg Trp Thr Thr Glu Gln Lys Leu Thr Ile Ile Glu Gln Ser
            20                  25                  30

Phe Glu Pro Gly Glu Thr Val Ser Ser Thr Ala Arg Arg His Gly Val
        35                  40                  45

Ala Pro Asn Leu Leu Tyr Arg Trp Arg Arg Leu Leu Ser Glu Gly Gly
    50                  55                  60

Ala Ala Ala Val Asp Ser Asp Glu Pro Val Val Gly Asn Ser Glu Val
65                  70                  75                  80

Lys Lys Leu Glu Asp Arg Val Arg Glu Leu Glu Arg Met Leu Gly Arg
                85                  90                  95

Lys Thr Met Glu Val Glu Ile Leu Arg Glu Ala Leu Ser Lys Ala Asp
            100                 105                 110

Ser Lys Lys Arg Ile Ser Arg Pro Ile Leu Leu Pro Lys Asp Gly Ser
        115                 120                 125

Arg

<210>  5
<211>  275
<212>  PRT
<213>  Agrobacterium tumefaciens

<400>  5

Met Lys Ala Val Ala Asp Thr Leu Gly Val Ser Arg Ser Asn Leu Ile
1               5                   10                  15

Glu Arg Leu Lys Gly Arg Ser Lys Pro Arg Gly Pro Tyr Asn Lys Ala
            20                  25                  30

Glu Asp Ala Glu Leu Leu Pro Ala Ile Arg Arg Leu Val Asp Gln Arg
        35                  40                  45

Pro Thr Tyr Gly Tyr Arg Arg Ile Ala Ala Leu Leu Asn Arg Glu Arg
    50                  55                  60

Arg Ala Ala Asp Gln Pro Val Val Asn Ala Lys Arg Val His Arg Ile
65                  70                  75                  80

Met Gly Asn His Ala Met Leu Leu Glu Lys His Thr Ala Val Arg Lys
                85                  90                  95

Gly Arg Leu His Asp Gly Lys Val Met Val Met Arg Ser Asn Leu Arg
            100                 105                 110

Trp Cys Ser Asp Gly Leu Glu Phe Ala Cys Trp Asn Gly Glu Val Ile
        115                 120                 125

Arg Leu Ala Phe Ile Ile Asp Ala Phe Asp Arg Glu Ile Ile Ala Trp
    130                 135                 140

Thr Ala Val Ala Asn Ala Gly Ile Ser Gly Ser Asp Val Arg Asp Met
145                 150                 155                 160

Met Leu Glu Ala Val Glu Lys Arg Phe His Ala Thr Arg Ala Pro His
                165                 170                 175

Ala Ile Glu His Leu Ser Asp Asn Gly Ser Ala Tyr Thr Ala Arg Asp
            180                 185                 190

Thr Arg Leu Phe Ala Gln Ala Leu Asn Leu Thr Pro Cys Phe Thr Pro
        195                 200                 205

Val Ala Ser Pro Gln Ser Asn Gly Met Ser Glu Ala Phe Val Lys Thr
    210                 215                 220

Leu Lys Arg Asp Tyr Ile Arg Ile Ser Ala Leu Pro Asp Ala Gln Thr
225                 230                 235                 240

Ala Leu Arg Leu Ile Asp Gly Trp Ile Glu Asp Tyr Asn Glu Ile His
                245                 250                 255

Pro His Ser Ala Leu Lys Met Ala Ser Pro Arg Gln Phe Ile Arg Ala
            260                 265                 270

Lys Ser Ile
        275

<210>  6
<211>  404
<212>  PRT
<213>  Agrobacterium tumefaciens

<400>  6

Met Ser Asn Asp Tyr Arg His Val Glu Leu Leu Thr Gly Asp Val Arg
1               5                   10                  15

Arg Arg Arg Trp Thr Thr Glu Gln Lys Leu Thr Ile Ile Glu Gln Ser
            20                  25                  30

Phe Glu Pro Gly Glu Thr Val Ser Ser Thr Ala Arg Arg His Gly Val
        35                  40                  45

Ala Pro Asn Leu Leu Tyr Arg Trp Arg Arg Leu Leu Ser Glu Gly Gly
    50                  55                  60

Ala Ala Ala Val Asp Ser Asp Glu Pro Val Val Gly Asn Ser Glu Val
65                  70                  75                  80

Lys Lys Leu Glu Asp Arg Val Arg Glu Leu Glu Arg Met Leu Gly Arg
                85                  90                  95

Lys Thr Met Glu Val Glu Ile Leu Arg Glu Ala Leu Ser Lys Ala Asp
            100                 105                 110

Ser Lys Lys Thr Asp Ile Ala Ala Asp Leu Val Ala Glu Gly Arg Phe
        115                 120                 125

Ala Met Lys Ala Val Ala Asp Thr Leu Gly Val Ser Arg Ser Asn Leu
    130                 135                 140

Ile Glu Arg Leu Lys Gly Arg Ser Lys Pro Arg Gly Pro Tyr Asn Lys
145                 150                 155                 160

Ala Glu Asp Ala Glu Leu Leu Pro Ala Ile Arg Arg Leu Val Asp Gln
                165                 170                 175

Arg Pro Thr Tyr Gly Tyr Arg Arg Ile Ala Ala Leu Leu Asn Arg Glu
            180                 185                 190

Arg Arg Ala Ala Asp Gln Pro Val Val Asn Ala Lys Arg Val His Arg
        195                 200                 205

Ile Met Gly Asn His Ala Met Leu Leu Glu Lys His Thr Ala Val Arg
    210                 215                 220

Lys Gly Arg Leu His Asp Gly Lys Val Met Val Met Arg Ser Asn Leu
225                 230                 235                 240

Arg Trp Cys Ser Asp Gly Leu Glu Phe Ala Cys Trp Asn Gly Glu Val
                245                 250                 255

Ile Arg Leu Ala Phe Ile Ile Asp Ala Phe Asp Arg Glu Ile Ile Ala
            260                 265                 270

Trp Thr Ala Val Ala Asn Ala Gly Ile Ser Gly Ser Asp Val Arg Asp
        275                 280                 285

Met Met Leu Glu Ala Val Glu Lys Arg Phe His Ala Thr Arg Ala Pro
    290                 295                 300

His Ala Ile Glu His Leu Ser Asp Asn Gly Ser Ala Tyr Thr Ala Arg
305                 310                 315                 320

Asp Thr Arg Leu Phe Ala Gln Ala Leu Asn Leu Thr Pro Cys Phe Thr
                325                 330                 335

Pro Val Ala Ser Pro Gln Ser Asn Gly Met Ser Glu Ala Phe Val Lys
            340                 345                 350

Thr Leu Lys Arg Asp Tyr Ile Arg Ile Ser Ala Leu Pro Asp Ala Gln
        355                 360                 365

Thr Ala Leu Arg Leu Ile Asp Gly Trp Ile Glu Asp Tyr Asn Glu Ile
    370                 375                 380

His Pro His Ser Ala Leu Lys Met Ala Ser Pro Arg Gln Phe Ile Arg
385                 390                 395                 400

Ala Lys Ser Ile

<210>  7
<211>  61
<212>  DNA
<213>  Agrobacterium tumefaciens

<400>  7
ttcatcacca tctatcaggc gctttcactg atgacgacct atgaatacat cctgctgatc      60

a                                                                      61

<210>  8
<211>  25
<212>  DNA
<213>  Agrobacterium tumefaciens

<400>  8
tggcaggata tattgtggtg taaac                                            25

<210>  9
<211>  25
<212>  DNA
<213>  Agrobacterium tumefaciens

<400>  9
tgacaggata tattggcggg taaac                                            25

<210>  10
<211>  30
<212>  DNA
<213>  Agrobacterium tumefaciens

<400>  10
gaggctggat atattgccgg tcgaagtggt                                       30